# EUROPÄISCHE PATENTANMELDUNG

(11) **EP 0 974 307 A1**
(43) Veröffentlichungstag der Anmeldung: **26.01.2000**
(21) Anmeldenummer: 99113857.9
(22) Anmeldetag: 15.07.1999
(51) Int. Cl.: A61B 18/14

(54) **Elektrode für ein elektrochirurgisches Handstück**

(30) Priorität: 21.07.1998 DE 29812998 U
(71) Anmelder: KALTENBACH & VOIGT GMBH & CO., 88400 Biberach (DE)
(72) Erfinder: Schmid, Gerhard, D-88441 Mittelbiberach (DE)
(74) Vertreter: Schmidt-Evers, Jürgen, Dipl.-Ing.

(57) **Zusammenfassung**

Die Erfindung betrifft eine Elektrode (3) für ein elektrochirurgisches Handstück (1), insbesondere zur Paradontalbehandlung, bestehend aus eimem zum Verbinden mit dem Handstück (1) bestimmten geraden Kupplungsabschnitt (4), einem Arbeitsendabschnitt (6) und einem zumindest teilweise gekrümmten Verbindungsschaft (5). Um handhabungsfreundliche Stellungen zu erreichen, liegt das Ende des Arbeitsendabschnittes (6) auf der gedachten Verlängerung des geraden Kupplungsabschnittes (4).

## Beschreibung

Die Erfindung bezieht sich auf eine Elektrode nach dem Oberbegriff des Anspruchs 1.

Bei einer Elektrode dieser Art handelt es sich um ein Spezialwerkzeug, in dessen engerem Bezirk im Funktionsbetrieb im Körpergewebe des menschlichen oder tierischen Körpers eine hohe spezifische Stromdichte erzeugt wird, die die erforderliche Erwärmung des Körpergewebes bewirkt, und z.B. zum Schließen von Gefäßen und zur Ausführung von Schnitten im weichen Körpergewebe benutzt wird. Bekannte Elektroden erstrecken sich gerade oder winkelförmig. Die letztere Form begünstigt den Blick des Behandlers auf die Behandlungsstelle und ist auch aus Handhabungsgründen vorteilhaft, da der Kupplungsabschnitt und ein damit verbundenes Handstück in einer sich von der Behandlungsstelle weg erstreckenden Position handhabungsfreundlich ergriffen und gehandhabt werden können. Es sind auch bereits Elektroden bekannt geworden, deren Kupplungsabschnitt quer bügelförmig ausgeformt ist und einen bezüglich der Mittelachse des Kupplungsabschnitts divergent verlaufenden Schenkel und einen sich daran anschließenden konvergent verlaufenden Schenkel aufweist, an den der Arbeitsendabschnitt eine Verlängerung bildet und jenseits einer Verlängerung der Mittelachse des Kupplungsabschnitts endet. Diese eingangs angegebene Ausgestaltung knüpft an bekannte Werkzeugformen von Zahnsteinentfernungsgeräten an und gewährleistet zwar handhabungsgünstige Stellungen der Elektrode im Funktionsbetrieb, jedoch ist die Bewegung und Führung während der Handhabung der Elektrode erschwert. Dies ist darauf zurückzuführen, daß im Funktionsbetrieb das vordere Ende des Arbeitsendabschnitts aufgrund seines Widerstandes, den es am Gewebe findet, ein Drehmoment auf das Handstück ausübt, das von einer gewollten Schnittbewegung ablenkt und das der Behandler zu korrigieren hat, wenn er die gewollte Schnittbewegung beibehalten will. Hierdurch wird die Handhabung erschwert, weil der Behandler eine größere Aufmerksamkeit auf die Handhabung richten muß. Zwar sind die entstehenden Kräfte gering, jedoch handelt es sich bei der Benutzung einer Elektrode um Feinarbeiten, bei denen auch geringe Störungen der Schnittführung die Handhabung erschweren.

Der Erfindung liegt die Aufgabe zugrunde, eine Elektrode der eingangs angegebenen Art so auszugestalten, daß bei Gewährleistung handhabungsgünstiger Stellungen eine leichte Bewegung und Führung während der Handhabung möglich ist.

Diese Aufgabe wird durch die Merkmale des Anspruchs 1 gelöst.

Bei der erfindungsgemäßen Ausgestaltung ist das freie Ende des Arbeitsendabschnitts auf einer gedachten Verlängerung des geraden Kupplungsabschnitts bzw. auf der Längsmittelachse des Kupplungsabschnitts angeordnet. In dieser Stellung übt der Arbeitsendabschnitt dann, wenn er im Funktionsbetrieb einen Arbeitswiderstand am Körpergewebe findet kein Drehmoment auf das Handstück auf, sondern es entstehen im wesentlichen lediglich Zug- oder Druckkräfte, je nachdem in welche axiale Richtung die Elektrode bewegt wird, oder seitliche Biegekräfte auf, wenn die Elektrode seitlich bewegt wird. Vorbeschriebene Drehmomente entstehen folglich nicht, und deshalb sind die Bewegung und Führung während der Handhabung und die Ausführung von Schnittbewegungen wesentlich erleichtert. Dagegen sind günstige Handhabungsstellungen weiterhin gewährleistet, da auch bei der erfindungsgemäßen Ausgestaltung die seitlich ausgeformte Form der Elektrode vorhanden ist.

Da für eine vorliegende Elektrode kein besonderer motorischer Bewegungsantrieb erforderlich ist, wie es z.B. bei einem Rotationswerkzeug der Fall ist, und außerdem bei der Behandlung in vielen Fällen Elektroden unterschiedlicher Form benutzt werden, die jeweils auszutauschen sind, ist zur Kupplung der Elektrode mit dem Handstück eine besonders einfache Steckverbindung entwickelt worden mit einer geschlitzten und elastisch aufweitbaren Klemmhülse im Handstück, in die der Kupplungsabschnitt mit leichtem Druck einschiebbar und zwecks Lösung der Elektrode wieder herausziehbar ist. Bei einer solchen Steckverbindung führen die vorbeschriebenen Drehmomente bei der bekannten Ausgestaltung dazu, daß die Elektrode in der Steckfassung verdreht wird, was eine zusätzlich manuelle Korrektur der Schnittführung erfordert und die Handhabung zusätzlich erschwert. Die erfindungsgemäße Ausgestaltung eignet sich deshalb insbesondere für eine Elektrode mit einem zylindrischen Kupplungsabschnitt bzw. für ein Instrument, das eine erfindungsgemäße Elektrode und ein Handstück mit der vorbeschriebenen Steckverbindung umfaßt.

In den Unteransprüchen sind Merkmale enthalten, die sich auf besondere Formen für die Elektrode oder deren Arbeitsendabschnitt beziehen, wobei die Eignung bezüglich handhabungsfreundlicher Arbeitsstellungen weiter verbessert wird und ein größerer Bewegungsfreiraum, insbesondere in der Längsrichtung der Elektrode geschaffen wird.

Nachfolgend werden die Erfindung und weitere durch sie erzielbare Vorteile anhand von Ausführungsbeispielen und Zeichnungen näher beschrieben. Es zeigen
- Fig. 1: ein medizinisches oder dentalmedizinisches Instrument mit einem Handstück und einer daran angekuppelten erfindungsgemäßen Elektrode in der Seitenansicht;
- Fig. 2: ein Instrument mit einer erfindungsgemäßen Elektrode in abgewandelter Ausgestaltung;
- Fig. 3: ein Instrument mit einer erfindungsgemäßen Elektrode in weiter abgewandelter Ausgestaltung;
- Fig. 4: ein Instrument mit einer erfindungsgemäßen Elektrode in weiter abgewandelter Ausgestaltung.

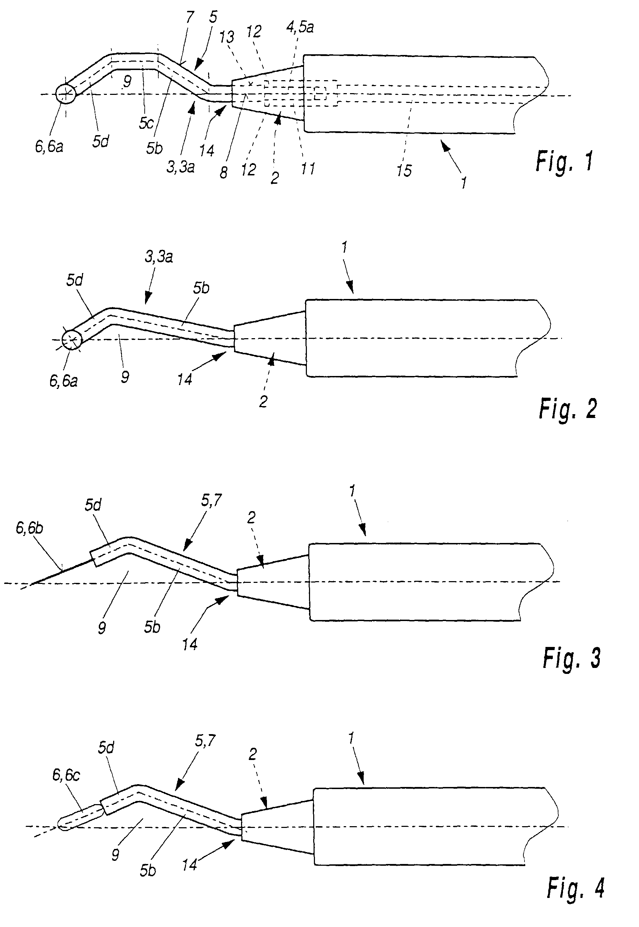

Die Hauptteile des Instruments sind ein stabförmiges Handstück 1 mit einer Kupplungsvorrichtung 2 in seinem vorderen Endbereich und eine stiftförmige Elektrode 3, bestehend aus einem ihren hinteren Endbereich bildenden zylindrischen Kupplungsabschnitt 4, an den sich nach vorne ein Verbindungsabschnitt 5 anschließt, an dessen vorderem Ende ein Arbeitsendabschnitt 6 angeordnet ist. Beim Ausführungsbeispiel nach Fig. 1 ist der Arbeitsendabschnitt 6 eine Kugel 6a, die an der Stirnfläche des Verbindungsschaftes 5 befestigt ist. Der Verbindungsabschnitt 5 ist von einer Isolationshülle 7 umgeben, die zugleich eine Wärmedämmschicht bildet. Der Kern des Verbindungsabschnittes 5 besteht aus einem elektrisch leitenden Material, insbesondere Metall, und kann durch einen vorzugsweise runden Draht gebildet sein, der im hinteren Endbereich den Kupplungsabschnitt 4 bildet und im übrigen Bereich von der Isolationshülle 7 umgeben ist.

Im vorderen Endbereich oder hier im Bereich der vorderen Hälfte des Verbindungsschaftes 5 ist dieser mit einer quer zur Längsmittelachse 8 gerichteten Krümmung 3a bügelförmig ausgeformt, so daß sich an einen ersten, koaxial zur Längsmittelachse 8 erstreckenden Verbindungsschaftabschnitt 5a ein sich nach vorne schräg und divergent erstreckender Verbindungsschaftabschnitt 5b anschließt, der mit einem etwa achsparallel versetzten Verbindungsschaftabschnitt 5c verbunden ist, von dessen vorderen Ende sich ein Verbindungsschaftabschnitt 5d schräg konvergent bis in den Bereich einer Verlängerung der Längsmittelachse 8 erstreckt, so daß die Kugel 6a sich in einer koaxialen Position befindet oder die Längsmittelachse 8 eine Tangente der Kugel 6a bildet oder die Kugel 6a schneidet. Die Verbindungsschaftabschnitte 5b, 5c und 5d begrenzen einen trapezförmigen Freiraum 9, in dem sich ein zugehöriger Abschnitt der Längsmittelachse 8 frei erstreckt.

Die Kupplungsvorrichtung 2 weist eine Klemmhülse 11 auf, die mit Ausnahme ihres hinteren Endbereichs ein- oder mehrfach radial geschlitzt ist, so daß sich einander gegenüberliegende Klemmsegmente 12 ergeben, und die insbesondere mit ihrem hinteren Ende so im Handstück, z.B. in einer Kammer, befestigt, z.B. eingebettet, ist, daß die Klemmsegmente 12 radial elastisch spreizbar sind. Der Innendurchmesser der Klemmhülse 11 ist etwas kleiner bemessen als der Durchmesser des Kupplungsabschnitts 4. Vor der Klemmhülse 11 weist das Handstück ein koaxiales rundes Loch 13 auf, dessen Durchmesser mit Bewegungsspiel dem Durchmesser der Isolationshülle 7 entspricht. Infolgedessen ist die Elektrode 3 mit ihrem Kupplungsabschnitt 4 von vorne in die Kupplungsvorrichtung 2 einsteckbar, wobei die Klemmsegmente 12 durch das Einschieben des Kupplungsabschnitts 4 gespreizt werden und dieser durch die elastische Rückstellkraft der Klemmsegmente 12 klemmend gehalten wird. Hierdurch ist eine Steckverbindung 14 gebildet, die handhabungsfreundlich und schnell durch Einstecken herbeigeführt und durch Herausziehen gelöst werden kann. Die Klemmkraft braucht nicht groß zu sein, da an der Elektrode 3 im Funktionsbetrieb keine großen axialen Kräfte oder auch Drehkräfte wirken.

Von hinten erstreckt sich eine elektrische Leitung 15 z.B koaxial bis zur Klemmhülse 11, mit der sie elektrisch leitend verbunden ist. Im Funktionsbetrieb ist die elektrische Leitung 15 an eine elektrische Stromquelle angeschlossen, der über die Klemmhülse 11 aus elektrisch leitendem Material und den Elektrodenkern zum Arbeitsendabschnitt 6 geleitet wird.

Die Klemmhülse 11 kann in einer Kammer einer Frontkappe des Handstücks 1 angeordnet sein, die von vorne aufsetzbar oder aufschraubbar mit dem übrigen Teil des Handstücks verbunden ist. Das Handstück 2 besteht aus elektrisch nicht leitendem Material, insbesondere Kunststoff.

Beim Ausführungsbeispiel nach Fig. 2, bei dem gleiche oder vergleichbare Teile mit gleichen Bezugszeichen versehen sind, unterscheidet sich von dem Ausführungsbeispiel nach Fig. 1 nur durch die Krümmung 3a bzw. bügelförmige Ausformung des Verbindungsschaftes 5. Gemäß Fig. 2 ist ein achsparalleler Verbindungsschaftabschnitt 5c fortgelassen, so daß die schräg bzw. divergenten und konvergenten Verbindungsschaftschenkel 5b, 5d im sich ergebenden Schenkelbereich unmittelbar miteinander verbunden sind und einen winkelförmigen Freiraum 9 begrenzen. Der Winkel, den die Verbindungsschaftabschnitte 5b, 5d einschließen, ist vorzugsweise kleiner als der Winkel, den die Verbindungsschaftabschnitte 5b, 5d gemäß Fig. 1 einschließen. Die Winkel, die die Verbindungsschaftabschnitte 5b, 5d mit der Mittelachse 8 einschließen, sind bei der Ausgestaltung nach Fig. 1 gleich und der Ausgestaltung nach Fig. 2 unterschiedlich, wobei der erste Winkel spitzer ist als der zweite Winkel.

Bei der Ausgestaltung nach Fig. 3, bei der gleiche oder vergleichbare Teile ebenfalls mit gleichen Bezugszeichen versehen sind, ist der Arbeitsendabschnitt 6 durch eine sich vorzugsweise gerade erstreckende Nadel 6b gebildet, wobei - im Vergleich mit der Ausgestaltung nach Fig. 2 - entweder nur der hintere Verbindungsschaftschenkel 5b vorhanden ist und die Nadel 6b am vorderen Ende des Verbindungsschaftschenkels 5b befestigt ist und zwar am Verbindungsschaftkern, oder die Nadel 6b an einem verkürzten Verbindungsschaftschenkel 5d befestigt ist. Dabei erstreckt sich die Nadel 6b etwa in der Schräglage des Verbindungsschaftschenkels 5d bezüglich der Längsmittelachse 8 konvergent nach vorne, wobei ihr vorderes Ende auf der Längsmittelachse 8 angeordnet ist und die mit der Längsmittelachse 8 eingeschlossenen Winkel gleich sein können.

Beim Ausführungsbeispiel nach Fig. 4, bei dem gleiche oder vergleichbare Teile ebenfalls mit gleichen Bezugszeichen versehen sind, ist der Arbeitsendabschnitt 6 durch eine Drahtschlinge 6c gebildet, die länglich ausgebildet ist, d.h. z.B. die Form eines flachgedrückten Kreises aufweist, jedoch auch andere Formen aufweisen kann, z.B. die Form eines Kreises oder eines Dreiecks oder einer Raute. Die Ebene der Drahtschlinge 6c erstreckt sich vorzugsweise in der Ebene der seitlichen Krümmung 3a bzw. bügelförmigen Ausformung, wobei die Größe der Drahtschlinge der Größe der Kugel 6a gem. Fig. 1 und 2 entsprechen kann, d.h. in etwa an die Querschnittsabmessung des Verbindungsschaftes 5 entsprechen kann und den vorderen Schenkel 5d der Ausformung teilweise ersetzen kann, wie es Fig. 4 zeigt, oder auch vollständig ersetzen kann. Der Durchmesser des Verbindungschaftes 5 kann z.B. etwa 2 bis 5 mm betragen, je nach Arbeitseinsatz und Leistungsanforderung.

Da bei den erfindungsgemäßen Ausgestaltungen der Arbeitsendabschnitt 6 sich jeweils auf der Längsmittelachse 8 befindet, werden im Funktionsbetrieb des länglichen Handstücks 1 bei in der Längsrichtung der Längsmittelachse 8 gerichteten Bewegungen des Handstücks 1 keine Drehmomente erzeugt, so daß auch keine daraus resultierenden, störenden Drehmomente am Handstück wirken können. Dies gilt auch für seitliche Bewegungen des Handstücks 1. Da keine Drehmomente entstehen, werden auch Verdrehungen der Elektrode 3 in der Steckfassung bzw. Klemmhülse 11 vermieden.

Eine erfindungsgemäße Elektrode 3 eignet sich sowohl für leicht als auch erschwert zugängliche Behandlungsstellen des menschlichen oder tierischen Körpers, unter anderem auch im Mundraum, insbesondere zur Paradontalbehandlung, wie z.B. mit der Ausgestaltung nach Fig. 3 oder 4.

## Patentansprüche

1. Elektrode (3) für ein elektrochirurgisches Handstück (1), insbesondere zur Paradontalbehandlung, bestehend aus einem zum Verbinden mit dem Handstück (1) bestimmten geraden Kupplungsabschnitt (4), einem Arbeitsendabschnitt (6) und einem zumindest teilweise gekrümmten Verbindungsschaft (5),
**dadurch gekennzeichnet,**
daß das Ende des Arbeitsendabschnittes (6) auf der gedachten Verlängerung des geraden Kupplungsabschnittes (4) liegt.

2. Elektrode nach Anspruch 1,
**dadurch gekennzeichnet,**
daß die Krümmung (3a) des Verbindungsschaftes (5) durch einen ersten, sich bezüglich der Längsmittelachse (8) schräg divergent erstreckenden Verbindungsschaftschenkel (5b) und einen sich daran anschließenden konvergent erstreckenden Verbindungsschaftschenkel (5d) gebildet ist.

3. Elektrode nach Anspruch 2,
**dadurch gekennzeichnet,**
daß sich zwischen den divergent und konvergent erstreckenden Verbindungsschaftschenkeln (5b, 5d) ein sich parallel zur Längsmittelachse (8) erstreckender Verbindungsschaftschenkel (5c) angeordnet ist.

4. Elektrode nach Anspruch 2 oder 3,
**dadurch gekennzeichnet,**
daß der vordere Verbindungsschaftschenkel (5d) kürzer bemessen ist, als der hintere Verbindungsschaftschenkel (5b).

5. Elektrode nach einem der Ansprüche 1 bis 3,
**dadurch gekennzeichnet,**
daß die Krümmung (3a) durch einen sich bezüglich der Längsmittelachse (8) divergent erstreckenden Verbindungsschaftschenkel (5b) gebildet ist und der Arbeitsendabschnitt (6) sich vom vorderen Ende des Verbindungsschaftschenkels (5b) bezüglich der Längsmittelachse (8) konvergent erstreckt.

6. Elektrode nach einem der vorherigen Ansprüche,
**dadurch gekennzeichnet,**
daß der Arbeitsendabschnitt (6) durch eine Kugel (6a) oder eine Nadel (6b) oder Drahtschlinge (6c) gebildet ist.

7. Elektrode nach einem der vorherigen Ansprüche,
**dadurch gekennzeichnet,**
daß sie mit einem Handstück (1), das in seinem vorderen Endbereich eine Kupplungsvorrichtung (2) aufweist, durch die die Elektrode (3) mit ihrem Kupplungsabschnitt (4) mit dem Handstück (2) verbindbar ist, ein Instrument bildet.

8. Elektrode nach Anspruch 7,
**dadurch gekennzeichnet,**
daß die Kupplungsvorrichtung (2) durch eine Steckverbindung (14) gebildet ist.

9. Elektrode nach Anspruch 8,
**dadurch gekennzeichnet,**
daß die Steckverbindung (14) für die Elektrode (3) koaxial von vorne zugänglich ist und vorzugsweise eine insbesondere radial geschlitzte Klemmhülse (11) aufweist, in die der Kupplungsabschnitt (4) einsteckbar ist.
